# EUROPEAN PATENT SPECIFICATION

(11) **EP 1 897 583 B1**
(45) Date of publication and mention of the grant of the patent: **04.08.2010**
(21) Application number: 07253495.1
(22) Date of filing: 04.09.2007
(51) Int. Cl.: A61M 25/09, A61B 1/01

(54) **Guidewire structure including a medical guidewire**
Führungsdrahtstruktur mit medizinischem Führungsdraht
Structure de fil guide avec fil guide médical

(30) Priority: 05.09.2006 US 515939
(43) Date of publication of application: 12.03.2008
(73) Proprietor: ETHICON ENDO-SURGERY, INC., Cincinnati, Ohio 45242 (US)
(72) Inventor: Spivey, James T., Loveland, Ohio 45140 (US); Bakos, Gregory J., Mason, Ohio 45040 (US)
(74) Representative: Tunstall, Christopher Stephen

(56) References cited:
- EP-A- 1 559 361
- FR-A- 2 850 285
- US-A1- 2002 156 454
- US-A1- 2005 101 836
- US-A1- 2005 256 429

## Description

**Field of the Invention**

The present invention is related generally to guidewire structures, and more particularly to a guidewire structure having a medical guidewire.

**Background of the Invention**

A physician typically accesses and visualizes tissue within a patient's gastrointestinal (GI) tract with an endoscope (such as a gastroscope or a colonoscope) having a long, flexible insertion tube. For the upper GI, a physician may insert a gastroscope into the sedated patient's mouth to examine and treat tissue in the esophagus, stomach, and proximal duodenum. For the lower GI, a physician may insert a colonoscope through the sedated patient's anus to examine the rectum and colon. Some endoscopes have a working channel in the insertion tube, typically about 2.5-3.5 millimeters in diameter, extending from a port in the handpiece to the distal portion of the insertion tube. A physician may insert medical devices into the working channel to help diagnose or treat tissue within the patient.

Guidewires have been used to aid the introduction of catheters (such as insertion tubes of endoscopes) and other instruments into many sites in the human body. US 2005/256429 A1, US 2005/101836 A1 and US 2002/156454 A1 disclose such guidewires. Many medical applications and specific designs of guidewires have been for cardiovascular use. There are, however, specific challenges relating to the use of guidewires in the GI tract, as opposed to the vascular system. Thus, the bowel is more tortuous, softer and generally of larger diameter. Furthermore, in the case of the small intestine and the colon, these are longer than most arteries or veins.

Still, scientists and engineers continue to seek improved guidewire structures having a medical guidewire.

**Summary**

The present invention provides a guidewire structure as claimed hereinafter.

Several benefits and advantages are obtained from one or more of the expressions of an embodiment of the invention. In one example, having a "non-sticky" overtube and having a loop-track or non-loop-track medical guidewire including a "sticky" first segment which can be slidably covered and slidably exposed by the overtube is expected to allow easier extension of the covered first segment in a body lumen of a patient followed by improved anchoring of the uncovered first segment against patient tissue resulting in improved advancement of an endoscope insertion tube along the anchored uncovered first segment.

**Brief Description of the Figures**

FIGURE 1 is a schematic side-elevational cutaway view of a first embodiment of a medical instrument having a catheter and employing an embodiment of a guidewire structure of the invention, wherein the guidewire structure has a medical guidewire and an overtube, wherein the medical guidewire is employed as a loop-track guidewire, wherein a shortened view of the entire working portion of the medical guidewire is shown extending beyond the distal end of the catheter, and wherein the overtube has been pulled to slidingly expose a first segment of the medical guidewire;

FIGURE 2 is a view as in Figure 1 but previous in time to Figure 1, wherein the overtube has been pushed to slidingly cover the first segment of the medical guidewire before the covered first segment was extended beyond the distal end of the catheter;

FIGURE 3 is a straightened side-elevational view of the working portion of the medical guidewire of Figure 1;

FIGURE 4 is a cross-sectional view of the first segment of the working portion of the medical guidewire of Figure 3 taken along lines 4-4 of Figure 3;

FIGURE 5 is a cross-sectional view of the second segment of the working portion of the medical guidewire of Figure 3 taken along lines 5-5 of Figure 3;

FIGURE 6 is a cross-sectional view of the guidewire structure of Figure 1 taken along lines 6-6 of Figure 1 showing the overtube surrounding a leg of the medical guidewire;

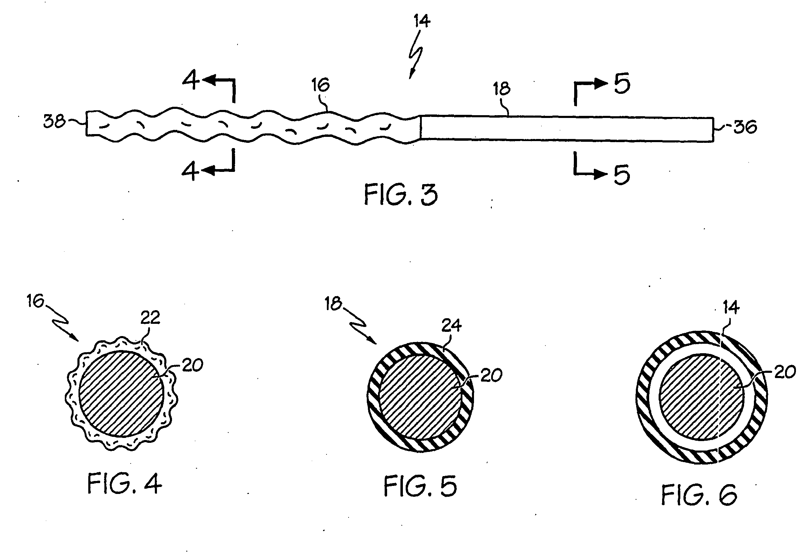

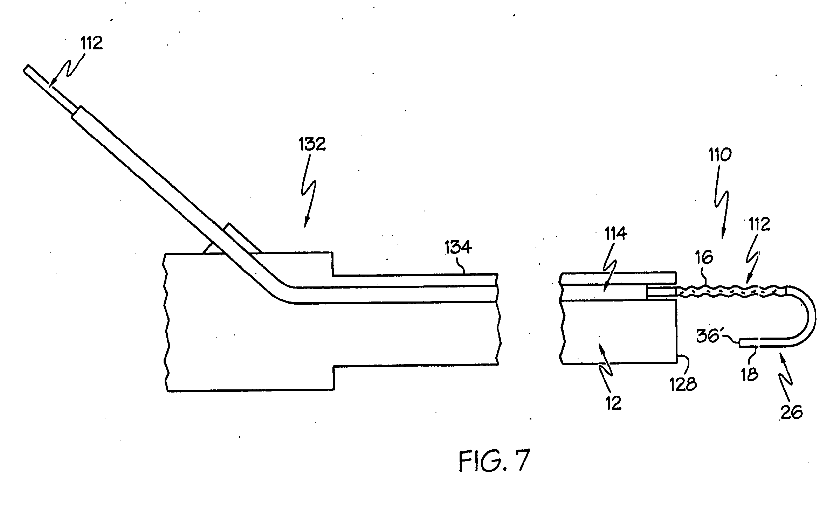

FIGURE 7 is a schematic side-elevational cutaway view of a second embodiment of a medical instrument having a catheter and employing an alternate embodiment of a guidewire structure of the invention, wherein the guidewire structure has a medical guidewire and an overtube, wherein the medical guidewire has the working portion of Figure 3 and is employed as a non-loop-track guidewire, wherein a shortened view of the entire working portion of the medical guidewire is shown extending beyond the distal end of the catheter, and wherein the overtube has been pulled to slidingly expose a first segment of the medical guidewire; and

FIGURE 8 is a view as in Figure 7 but previous in time to Figure 7, wherein the overtube has been pushed to slidingly cover the first segment of the medical guidewire before the covered first segment was extended beyond the distal end of the catheter.

**Detailed Description**

Before explaining the several embodiments of the present invention in detail, it should be noted that each embodiment is not limited in its application or use to the details of construction and arrangement of parts and steps illustrated in the accompanying drawings and description. The illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention.

It is further understood that any one or more of the following-described embodiments, examples, etc. can be combined with any one or more of the other following-described embodiments, examples, etc.

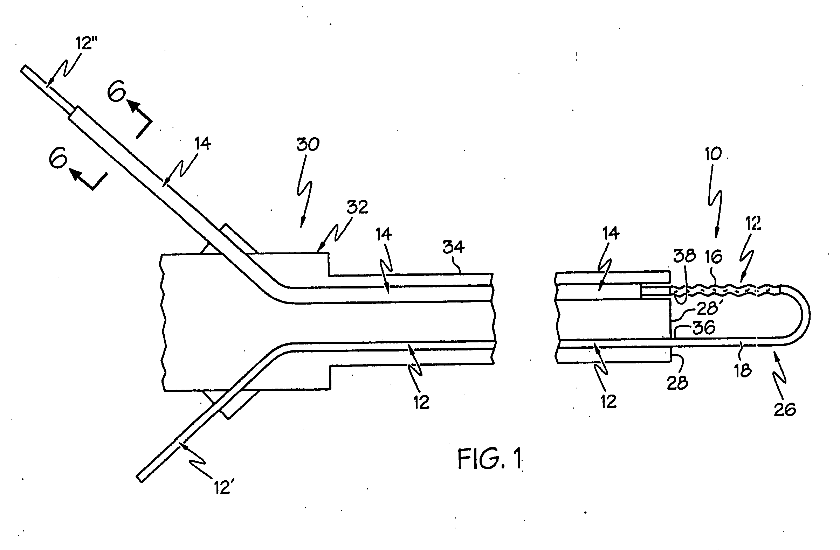

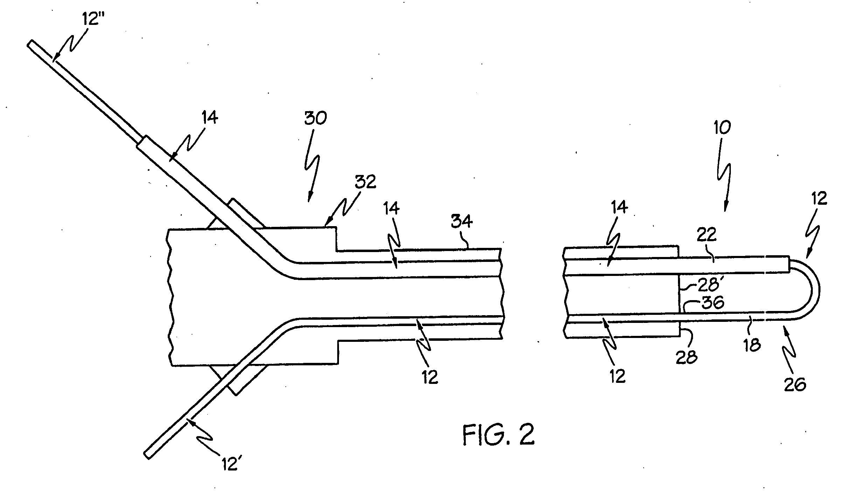

An embodiment of a guidewire structure 10 of the invention is shown in Figures 1-6. A first expression of the guidewire structure 10 of the embodiment of Figures 1-6 includes a medical guidewire 12 and an overtube 14. The medical guidewire 12 includes a first segment 16 and a lengthwise-adjoining second segment 18. The overtube 14 is adapted to slidably cover the first segment 16 (as shown in Figure 2) and to slidably expose the first segment (as shown in Figure 1). A minimum force required to slide the exposed first segment 16 over patient tissue is greater than a minimum force required to slide the covered first segment 16 over the patient tissue.

It is noted that the exposed first segment 16 when slidingly pushed over patient tissue sticks more to the patient tissue than does the covered first segment 16 when likewise slidingly pushed over the patient tissue. In one example, a minimum force required to slide the exposed first segment 16 over the patient tissue is greater than a minimum force required to slide the (exposed) second segment 18 over the patient tissue. It is also noted that the exposed first segment when slidingly pushed over the patient tissue sticks more to the patient tissue than does the (exposed) second segment 18 when likewise slidingly pushed over the patient tissue.

In one enablement of the first expression of the embodiment of Figures 1-6, the overtube 14 is flexible. In one variation, the medical guidewire 12 is resiliently flexible. In one modification, each of the first and second segments 16 and 18 is resiliently flexible.

In one construction of the first expression of the embodiment of Figures 1-6, the first segment 16 includes a first length of a core wire 20 and a mesh 22 surrounding, and attached to, the first length of the core wire 20. In one method, the mesh 22 is attached to the first length of the core wire 20 by an adhesive. In another method, not shown, a thin wall sleeve surrounds the mesh and is crimped against the core wire to trap the mesh between the sleeve and the core wire. In a further method, a heat shrinkable material surrounds the mesh and is heat shrunk against the core wire to trap the mesh between the sleeve and the core wire. Other methods are left to the artisan. In one example, the core wire 20 consists essentially of a monolithic length of a super-elastic alloy such as nitinol available from Nitinol Devices & Components (Fremont, CA). In the same or a different example, the mesh 22 consists essentially of polypropylene such as Gynemesh^{®} surgical mesh available from Johnson & Johnson Corporation (New Brunswick, NJ). In the same or a different example, the overtube 14 is a lubricious overtube such as one consisting essentially of Polytetrafluoroethylene (PTFE), such as Teflon^{®} PTFE available from Zeus, Inc (Orangeburg, SC). It is noted that the mesh 22 sticks to patient tissue more than does the overtube 14. In non-mesh constructions, not shown, the first segment has a shape (such as a corrugated shape), a texture, a surface roughness (such as that of a pitted or sandblasted surface), or a series of projections (such as bristles) that tend to grip onto tissue.

In the same or a different construction, the second segment 18 consists essentially of a second length of the core wire 20 and a lubricious sleeve 24 surrounding, and attached to, the second length of the core wire 20. In one variation, the first and second lengths are portions of a monolithic length of the core wire 20. Examples of materials for the lubricious sleeve 24 include, without limitation, Polytetrafluoroethylene (PTFE), such as Striped Teflon^{®} PTFE available from Zeus, Inc (Orangeburg, SC). In one method, the lubricious sleeve 24 is applied over the second length of the core wire 20 through a heat-shrink process well known in the art. It is noted that the mesh 22 sticks to patient tissue more than does the lubricious sleeve 24.

A second expression of the guidewire structure 10 of the embodiment of Figures 1-6 includes a medical guidewire 12 and an overtube 14. The medical guidewire 12 includes a working portion 26 which is extendable beyond a distal end 28 of a medical instrument 30. The working portion 26 includes a first segment 16 and a lengthwise-adjoining second segment 18. The overtube 14 surrounds the medical guidewire 12 and is adapted to slidably cover the first segment 16 (as shown in Figure 2) and to slidably expose the first segment (as shown in Figure 1). A minimum force required to slide the exposed first segment 16 over patient tissue is greater than a minimum force required to slide the covered first segment 16 over the patient tissue.

It is noted that the working portion 26 is a maximum portion of the medical guidewire 12 which can be extended beyond the distal end 28 of the medical instrument 30. Some applications of the guidewire structure 10 may require the entire working portion 26 to be extended beyond the distal end 28 while other applications may require less than the entire working portion 26 to be extended beyond the distal end 28. It is also noted that in some applications, the medical guidewire 12 is manually pushed (as intended by Figures 1 and 2) to extend at least some of the working portion 26 beyond the distal end 28, that in other applications a hand crank (not shown) is used to extend at least some of the working portion 26, and that in still other applications a motor (not shown) is used to extend at least some of the working portion 26. It is further noted that the examples, enablements, constructions, etc. of the first expression of the embodiment of Figures 1-6 are equally applicable to the second expression of the embodiment of Figures 1-6.

In one application of the second expression of the embodiment of Figures 1-6, the medical instrument 30 is an endoscope 32 having a flexible insertion tube 34. In this application, the distal end 28 of the medical instrument is a distal end 28' of the insertion tube 34. In one variation, the working portion 26 is extendable beyond the distal end 28' of the insertion tube 34 from within the insertion tube 34.

In a first deployment of the second expression of the embodiment of Figures 1-6, the working portion 26 is extendable as a loop track (as shown in Figures 1 and 2) beyond the distal end 28' of the insertion tube 34. Here, the length of the working portion 26 is a loop-track length of the working portion 26. In one construction, the loop-track length of the working portion 26 is at least six feet, and the working portion 26 has a substantially circular cross-section having a maximum diameter which is always less than 1,27 mm (0.050-inch) and a minimum diameter which is always at least 0,254 mm (0.010-inch).

In a first arrangement of the second expression of the embodiment of Figures 1-6, the working portion 26 extends as a loop track, the medical guidewire 12 includes a first leg 12' monolithically attached to and extending from a first end 36 of the working portion 26 (which is a proximal end of the second segment 18) proximally through a first passageway of the insertion tube 34 and outside the endoscope 32, and the medical guidewire 12 includes a second leg 12" monolithically attached to and extending from a second end 38 of the working portion 14 (which is a proximal end of the first segment 16) proximally through a second passageway of the insertion tube 34 and outside the endoscope 32. In a second arrangement, not shown, the first and second legs 12' and 12" extend through a single passageway such as a working channel of the insertion tube. In a third arrangement, not shown, the loop track extends beyond the distal end of the insertion tube from outside the exterior surface of the insertion tube with the first and/or second legs engaged by guide ways on the exterior surface of the insertion tube. Other arrangements are left to the artisan.

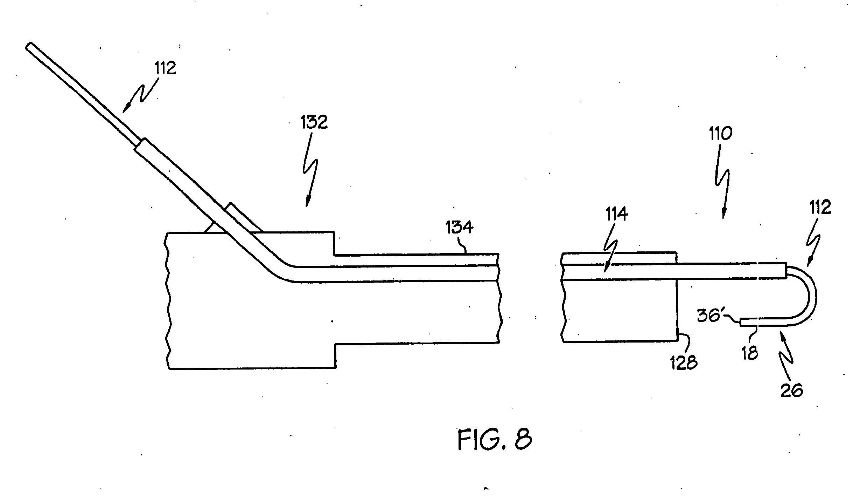

In a second deployment (shown in the alternate embodiment of Figures 7-8), a guidewire structure 110 includes a medical guidewire 112 having the working portion 26 shown in Figure 3, but the guidewire structure 110 is employed as a non-loop-track in a different endoscope 132 having an insertion tube 134. Here, the second segment 18 has a free end 36' which extends beyond the distal end 128 of the insertion tube 134 when the working portion 26 is extended beyond the distal end 128 of the insertion tube 134 The first segment 16 is exposed in Figure 7 and is covered by the overtube 114 in Figure 8.

In a different embodiment of the guidewire structure, not shown, the working portion of the medical guidewire consists essentially of the first segment. In one deployment, the working portion is a loop-track working portion. In a different deployment, the working portion is a non-loop-track working portion.

An exemplary method is for using a guidewire structure 10. The guidewire structure 10 includes a working portion 26 which is extendable beyond a distal end 28' of an insertion tube 34 of an endoscope 32, wherein the working portion 26 includes a medical guidewire 12 and an overtube 14. The medical guidewire 12 includes a first segment 16 and a lengthwise-adjoining second segment 18. The overtube 14 is adapted to slidably cover the first segment 16 and to slidably expose the first segment 16. A minimum force required to slide the exposed first segment 16 over patient tissue is greater than a minimum force required to slide the covered first segment 16 over the patient tissue, and a minimum force required to slide the exposed first segment 16 over the patient tissue is greater than a minimum force required to slide the second segment 18 over the patient tissue. The method includes steps a) through e). Step a) includes inserting the distal end 28' of the insertion tube 34 an initial distance into a body lumen of a patient. Step b) includes extending at least a portion of the second segment 18 beyond the distal end 28' of the insertion tube 34. Step c) includes extending at least a portion of the first segment 16 beyond the distal end 28' of the insertion tube 34 with the overtube 14 covering the extended first segment 16. Step d) includes sliding the overtube 14 off the extended first segment 16 exposing the extended first segment 16. Step e) includes advancing the insertion tube 34 along the exposed and extended first segment 16 further into the body lumen of the patient.

In one implementation of the method, step c) includes manually pulling the overtube 14 slidingly off the extended first segment 16. In a different implementation, step c) includes using a motor to pull the overtube slidingly off the extended first segment.

Several benefits and advantages are obtained from one or more of the embodiments of the invention. In one example, having a "non-sticky" overtube and having a loop-track or non-loop-track medical guidewire including a "sticky" first segment which can be slidably covered and slidably exposed by the overtube is expected to allow easier extension of the covered first segment in a body lumen of a patient followed by improved anchoring of the uncovered first segment against patient tissue resulting in improved advancement of an endoscope insertion tube along the anchored uncovered first segment.

While the present invention has been illustrated by descriptions of a method, several expressions of embodiments, and examples, etc. thereof, it is not the intention of the applicants to restrict or limit the scope of the appended claims to such detail. Numerous other variations, changes, and substitutions will occur to those skilled in the art without departing from the scope of the invention. It will be understood that the foregoing description is provided by way of example, and that other modifications may occur to those skilled in the art without departing from the scope of the appended Claims.

## Claims

1. A guidewire structure (10) comprising
a medical guidewire (12), wherein the medical guidewire (12) includes a first Segment (16) and a lengthwise-adjoining second segment (18),
**characterized by**
an overtube (14), wherein the overtube (14) is adapted to slidably cover the first segment (16) and to slidably expose the first segment (16), and wherein a minimum force required to slide the exposed first segment over patient tissue is greater than a minimum force required to slide the covered first segment over the patient tissue.

2. The guidewire structure of claim 1, wherein a minium force required to slide the exposed first segment over the patient tissue is greater than a minimum force required to slide the second segment over the patient tissue.

3. The guidewire structure of claim 2, wherein the first segment includes a first length of a core wire (20) and a mesh (22) surrounding, and attached to, the first length of the core wire.

4. The guidewire structure of claim 3, wherein the first length of the core wire consists essentially of nitinol and wherein the mesh consists essentially of polypropylene.

5. The guidewire structure of claim 3, wherein the overtube is a lubricious overtube.

6. The guidewire structure of claim 3, wherein the second segment consists essentially of a second length of the core wire (20) and a lubricious sleeve (24) surrounding, and attached to, the second length of the core wire.

7. The guidewire structure of claim 6, wherein the overture is a lubricious overtube.

8. A guidewire structure of any preceding claim, wherein the medical guidewire includes a working portion (26) which is extendable beyond a distal end (28) of a medical instrument (30), wherein the working portion includes the first segment and lengthwise-adjoining second segment.

9. The guidewire structure of claim 8, wherein the medical instrument is an endoscope having a flexible insertion tube (34), wherein the distal end of the medical instrument is a distal end (28') of the insertion tube and wherein the working portion is extendable beyond the distal end of the insertion tube from within the insertion tube.

10. The guidewire structure of claim 9, wherein the working portion is extendable as a loop-track beyond the distal end of the insertion tube.

11. The guidewire structure of claim 9, wherein the second segment has a free end which extends beyond the distal end of the insertion tube when the working portion is extended beyond the distal end of the insertion tube.

## Patentansprüche

1. Führungsdrahtstruktur (10), welche Folgendes umfasst:
einen medizinischen Führungsdraht (12), wobei der medizinische Führungsdraht (12) ein erstes Segment (16) und ein sich längsseitig anschließendes zweites Segment (18) aufweist,
**gekennzeichnet durch**
einen Übertubus (14), wobei der Übertubus (14) eingerichtet ist, um das erste Segment (16) verschiebbar zu verdecken und das erste Segment (16) verschiebbar freizulegen, und wobei eine minimale Kraft, welche erforderlich ist, um das freigelegte erste Segment über Patientengewebe zu schieben, größer ist als eine minimale Kraft, welche erforderlich ist, um das verdeckte erste Segment über das Patientengewebe zu schieben.

2. Führungsdrahtstruktur nach Anspruch 1, wobei eine minimale Kraft, welche erforderlich ist, um das freigelegte erste Segment über das Patientengewebe zu schieben, größer ist als eine minimale Kraft, welche erforderlich ist, um das zweite Segment über das Patientengewebe zu schieben.

3. Führungsdrahtstruktur nach Anspruch 2, wobei das erste Segment eine erste Länge eines Kerndrahtes (20) und ein Gittergewebe (22) aufweist, welches die erste Länge des Kerndrahtes umgibt und daran befestigt ist.

4. Führungsdrahtstruktur nach Anspruch 3, wobei die erste Länge des Kerndrahtes im Wesentlichen aus Nitinol besteht und wobei das Gittergewebe im Wesentlichen aus Polypropylen besteht.

5. Führungsdrahtstruktur nach Anspruch 3, wobei der Übertubus ein gleitfähiger Übertubus ist.

6. Führungsdrahtstruktur nach Anspruch 3, wobei das zweite Segment im Wesentlichen aus einer zweiten Länge des Kerndrahtes (20) und einer gleitfähigen Hülse (24) besteht, welche die zweite Länge des Kerndrahtes umgibt und daran befestigt ist.

7. Führungsdrahtstruktur nach Anspruch 6, wobei der Übertubus ein gleitfähiger Übertubus ist.

8. Führungsdrahtstruktur nach einem der vorhergehenden Ansprüche, wobei der medizinische Führungsdraht einen Arbeitsbereich (26) aufweist, welcher über ein distales Ende (28) eines medizinischen Instrumentes (30) hinaus ausfahrbar ist, wobei der Arbeitsbereich das erste Segment und das sich längsseits anschließende zweite Segment aufweist.

9. Führungsdrahtstruktur nach Anspruch 8, wobei das medizinische Instrument ein Endoskop ist, welches einen flexiblen Insertionstubus (34) aufweist, wobei das distale Ende des medizinischen Instruments ein distales Ende (28') des Insertionstubus ist und wobei der Arbeitsbereich von innerhalb des Insertionstubus über das distale Ende des Insertionstubus hinaus ausfahrbar ist.

10. Führungsdrahtstruktur nach Anspruch 9, wobei der Arbeitsbereich als eine schlingenförmige Bahn über das distale Ende des Insertionstubus hinaus ausfahrbar ist.

11. Führungsdrahtstruktur nach Anspruch 9, wobei das zweite Segment ein freies Ende aufweist, welches sich über das distale Ende des Insertionstubus hinaus erstreckt, wenn der Arbeitsbereich über das distale Ende des Insertionstubus hinaus ausgefahren ist.

## Revendications

1. Structure de fil guide (10) comprenant :
un fil guide médical (12) dans lequel le fil guide médical (12) comprend un premier segment (16) et un second segment contigu dans le sens de la longueur (18),
**caractérisée par**
un sur-tube (14) dans lequel le sur-tube (14) est adapté pour couvrir de manière coulissante le premier segment (16) et pour exposer de manière coulissante le premier segment (16) et dans lequel une force minimale requise pour faire coulisser le premier segment exposé sur le tissu du patient est supérieure à une force minimale requise pour faire coulisser le premier segment recouvert sur le tissu du patient.

2. Structure de fil guide selon la revendication 1, dans laquelle une force minimale requise pour faire coulisser le premier segment exposé sur le tissu du patient est supérieure à une force minimale requise pour faire coulisser le second segment sur le tissu du patient.

3. Structure de fil guide selon la revendication 2, dans laquelle le premier segment comprend une première longueur de fil central (20) et une maille (22) entourant, et fixée à, la première longueur du fil central.

4. Structure de fil guide selon la revendication 3, dans laquelle la première longueur du fil central est essentiellement constituée de nitinol et dans laquelle la maille est essentiellement constituée de polypropylène.

5. Structure de fil guide selon la revendication 3, dans laquelle le sur-tube est un sur-tube lubrifié.

6. Structure de fil guide selon la revendication 3, dans laquelle le second segment est constitué essentiellement d'une seconde longueur du fil central (20) et d'un manchon lubrifié (24) entourant, et fixé à, la seconde longueur du fil central.

7. Structure de fil guide selon la revendication 6, dans laquelle le sur-tube est un sur-tube lubrifié.

8. Structure de fil guide selon l'une quelconque des revendications précédentes, dans laquelle le fil guide médical comprend une partie de fonctionnement (26) qui est extensible au-delà d'une extrémité distale (28) d'un instrument médical (30), dans lequel la partie de fonctionnement comprend le premier segment et le second segment contigu dans le sens de la longueur.

9. Structure de fil guide selon la revendication 8, dans laquelle l'instrument médical est un endoscope ayant un tube d'insertion flexible (34), dans lequel l'extrémité distale de l'instrument médical est une extrémité distale (28') du tube d'insertion et dans lequel la partie de fonctionnement est extensible au-delà de l'extrémité distale du tube d'insertion depuis l'intérieur du tube d'insertion.

10. Structure de fil guide selon la revendication 9, dans laquelle la partie de fonctionnement est extensible sous la forme d'une boucle au delà de l'extrémité distale du tube d'insertion.

11. Structure de fil guide selon la revendication 9, dans laquelle le second segment a une extrémité libre qui s'étend au-delà de l'extrémité distale du tube d'insertion quand la partie de fonctionnement est étendue au-delà de l'extrémité distale du tube d'insertion.
